(19) Europäisches Patentamt / European Patent Office / Office européen des brevets

(11) EP 3 810 077 B1

(12) FASCICULE DE BREVET EUROPEEN

(45) Date de publication et mention de la délivrance du brevet:
**17.04.2024 Bulletin 2024/16**

(21) Numéro de dépôt: **19731742.3**

(22) Date de dépôt: **20.06.2019**

(51) Classification Internationale des Brevets (IPC):
***A61K 8/41*** *(2006.01)* ***A61Q 5/10*** *(2006.01)*
***A61K 8/73*** *(2006.01)* ***A61K 8/19*** *(2006.01)*

(52) Classification Coopérative des Brevets (CPC):
**A61K 8/41; A61K 8/19; A61K 8/73; A61Q 5/10**

(86) Numéro de dépôt international:
**PCT/EP2019/066369**

(87) Numéro de publication internationale:
**WO 2019/243512 (26.12.2019 Gazette 2019/52)**

# (54) COMPOSITION DE COLORATION CAPILLAIRE COMPRENANT UN COLORANT D'OXYDATION, UNE GOMME DE SCLÉROGLUCANE, UNE ALCALNOLAMINE ET UN AGENT ALCALIN MINÉRAL

HAARFÄRBEZUSAMMENSETZUNG MIT EINEM OXIDATIONSFARBSTOFF, EINEM SCLEROGLUCANGUMMI, EINEM ALCANOLAMIN UND EINEM MINERALISCHEN ALKALISCHEN MITTEL

HAIR DYEING COMPOSITION COMPRISING AN OXIDATION DYE, A SCLEROGLUCAN GUM, AN ALCANOLAMINE AND A MINERAL ALKALINE AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2018 FR 1855431**

(43) Date de publication de la demande:
**28.04.2021 Bulletin 2021/17**

(73) Titulaire: **L'OREAL**
**75008 Paris (FR)**

(72) Inventeurs:
- **MULLER, Sabrina**
  **93400 SAINT-OUEN (FR)**
- **CHARRIER, Delphine**
  **93400 SAINT-OUEN (FR)**
- **MILIC, Mladen**
  **93400 SAINT-OUEN (FR)**
- **YADEL, Cindy**
  **93400 SAINT-OUEN (FR)**
- **CARDONNEL, Fanny**
  **93400 SAINT-OUEN (FR)**

(74) Mandataire: **L'Oreal**
**Service D.I.P.I.**
**9, rue Pierre Dreyfus**
**92110 Clichy (FR)**

(56) Documents cités:
**US-A1- 2010 192 969** **US-A1- 2011 203 605**
**US-A1- 2011 209 720**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 810 077 B1

## Description

La présente invention concerne une composition pour la coloration des fibres kératiniques, en particulier des fibres kératiniques humaines telles que les cheveux.

L'invention concerne également un procédé de coloration mettant en oeuvre l'application de ladite composition sur les fibres kératiniques en particulier des fibres kératiniques humaines telles que les cheveux, ainsi qu'un dispositif à plusieurs compartiments, approprié pour la mise en oeuvre de ladite composition de coloration.

La présente invention a trait au domaine de la coloration des fibres kératiniques et plus particulièrement à celui de la coloration capillaire.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

La variété des molécules mises en jeu au niveau des bases d'oxydation et des coupleurs permet l'obtention d'une riche palette de couleurs.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

La coloration d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Le procédé de coloration doit également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine, afin d'obtenir une coloration la plus homogène possible des fibres kératiniques. Les compositions de coloration doivent également permettre de conférer de bonnes propriétés cosmétiques aux fibres kératiniques, en particulier du soin, de la douceur et/ou de la discipline, présenter de bonnes qualités d'usage, en particulier être faciles à appliquer, tout en permettant d'atteindre des résultats couleur visibles (c'est-à-dire notamment intenses, chromatiques), homogènes et tenaces.

Les compositions mises en oeuvre dans un procédé de coloration doivent, en outre, présenter de bonnes propriétés de mélange et d'application sur les fibres kératiniques, et notamment de bonnes propriétés rhéologiques pour ne pas couler, lors de leur application, sur le visage, le cuir chevelu, ou en dehors des zones que l'on se propose de teindre, cela permet notamment une application homogène des racines jusqu'au pointes.

La composition selon l'invention présente également une très bonne stabilité dans le temps pendant plusieurs semaines.

En particulier on cherche à obtenir des compositions de coloration stables dans le temps pendant plusieurs semaines. Par « stable » au sens de la présente invention on entend en particulier que des caractéristiques physiques telles que l'aspect, le pH et/ou la viscosité varient peu, voire ne varie pas, au court du temps, en particulier, que la viscosité de la composition n'évolue pas ou peu pendant le stockage et/ou que la composition ne déphase pas pendant le stockage.

En effet, il est souhaitable que les compositions de coloration soient stables dans le temps, en particulier stables après 1 mois à 45°C, voire après 2 mois à 45°C.

On cherche également à obtenir des compositions de coloration stables sur une large gamme de pH et en particulier vis-à-vis de pH extrêmes, par exemple à des pH alcalins allant de 9 à 12. Enfin, les compositions de coloration peuvent parfois être déstabilisées (déphasées) par des teneurs élevés en certains composés, comme par exemple en colorants d'oxydation et/ou des composés cationiques tels que des polymères cationiques, et il est donc souhaitable que ces compositions soient stables dans ces conditions, en particulier qui ne déphase pas.

US 2010/192969 A1 (DEGEORGE MICHAEL [US] ET AL) 5 août 2010 décrit une composition de coloration capillaire comprenant un agent épaississant.

Ces buts et d'autres sont atteints par la présente invention qui a donc pour objet une composition prête à l'emploi selon la revendication 1 pour la coloration d'oxydation des fibres kératiniques en particulier des fibres kératiniques humaines telles que les cheveux obtenue après mélange d'une composition (A) comprenant:

un ou plusieurs colorant(s) d'oxydation ;

une ou plusieurs gommes de scléroglucane, en une teneur totale supérieure ou égale à 0,5% en poids, par rapport au poids de la composition ;

un ou plusieurs agent(s) alcalin(s) minéral(aux) ; et
un ou plusieurs agent(s) alcalin(s) organique(s) choisi(s) parmi les alcanolamine(s) ;
et d'une composition (B) comprenant un ou plusieurs agent(s) oxydant(s) chimique(s).

Par « composition prête à l'emploi », on entend au sens de l'invention, toute composition destinée à être appliquée immédiatement sur les fibres kératiniques.

L'invention a également pour objet un dispositif (ou " kit ") à plusieurs compartiments permettant la mise en oeuvre de la composition de coloration des fibres kératiniques, de préférence comprenant au moins deux compartiments, un premier compartiment contenant la composition colorante (A) telle que définie précédemment, et le second compartiment contenant au moins une composition oxydante (B) comprenant au moins un agent oxydant chimique, de préférence choisi parmi le peroxyde d'hydrogène et/ou un ou plusieurs système(s) générateur(s) de peroxyde d'hydrogène, de préférence parmi le peroxyde d'hydrogène, les compositions des compartiments étant destinées à être mélangées avant application, pour donner la formulation après mélange,; en particulier le kit peut être un dispositif aérosol.

Au sens de la présente invention, par « composition pour la coloration » ou par « composition colorante », on entend une composition destinée à être appliquée sur les fibres kératiniques, de préférence les fibres kératiniques humaines et en particulier les cheveux, éventuellement après mélange avec une composition oxydante comprenant au moins un agent oxydant chimique. Au sens de la présente invention, par « composition colorante prête à l'emploi » ou « composition prête à l'emploi », on entend une composition résultant du mélange d'une composition colorante et d'une composition oxydante. La composition colorante prête à l'emploi peut être préparée juste avant l'application sur lesdites fibres kératiniques.

Les compositions selon l'invention permettent ainsi de conduire à de très bonnes performances de coloration des fibres kératiniques, notamment en termes de montée, d'intensité, de chromaticité et/ou de sélectivité. Elles permettent également d'obtenir des compositions ayant de bonnes propriétés rhéologiques pour ne pas couler, lors de leur application, sur le visage, le cuir chevelu, ou en dehors des zones que l'on se propose de teindre.

Les compositions selon l'invention sont stables. Par « stable » au sens de la présente invention on entend en particulier que des caractéristiques physiques telles que l'aspect, le pH et/ou la viscosité varient peu, voire ne varie pas, au cours du temps, en particulier, que la viscosité de la composition n'évolue pas ou peu pendant le stockage et/ou que la composition ne déphase pas au cours du stockage. En particulier, il est souhaitable que les compositions de coloration soient stables dans les temps, en particulier stables après 1 mois à 45°C, voire après 2 mois à 45°C.

De plus les compositions selon l'invention présentent l'avantage d'être stables (ne déphasent pas) indépendamment du pH et en particulier vis-à-vis de pH extrêmes, notamment à pH alcalin supérieur à ou égal à 9, par exemple à des pH alcalins allant de 9 à 12. Enfin, les compositions sont de préférence stables (ne déphasent pas) même en présence d'une teneur importante en certains composés, comme par exemple en colorants d'oxydation et/ou des composés cationiques tels que des polymères cationiques.

Par ailleurs, les compositions de l'invention sont de façon avantageuse translucides, ce qui leur confère un aspect visuel esthétique et attrayant pour le consommateur. -

En particulier, les compositions selon l'invention permettent d'obtenir de très bonnes propriétés tinctoriales tout en diminuant les odeurs désagréables par rapport aux compositions de coloration classiques.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine, notamment dans les expressions « compris entre » et « allant de... à ... » et « variant de ... à ... » .

Les fibres kératiniques sont de préférence des fibres kératiniques humaines,de préférence les cheveux.

L'expression « *au moins un* » est équivalente à l'expression « *un ou plusieurs* ».

Avantageusement, la composition selon l'invention présente une texture épaissie, sous forme de crème ou de gel, de préférence de gel, de préférence elle est translucide.

Ainsi, la composition selon l'invention présente généralement à température ambiante (25°C) une viscosité supérieure à 50 cps, de préférence comprise entre 200 et 100000 cps, plus préférentiellement entre 500 et 50000 cps, et encore plus préférentiellement entre 800 à 10000 cps, mieux de 1000 à 8000 cps mesurée à 25°C à une vitesse de rotation de 200 tours/mn à l'aide d'un rhéomètre tel que le rheomat RM 180 équipé d'un mobile n° 3 ou 4, la mesure étant effectuée après 60 secondes de rotation du mobile (temps au bout duquel on observe une stabilisation de la viscosité et de la vitesse de rotation du mobile.

***Colorants d'oxydation***

La composition (A) selon l'invention comprend un ou plusieurs colorants d'oxydation.

Les précurseurs de colorant d'oxydation utilisables dans la présente invention sont en général choisis parmi les bases d'oxydation, éventuellement combinées à un ou plusieurs coupleurs.

Les bases d'oxydation peuvent être de préférence choisies parmi les paraphénylènediamines, les bis-phénylalkylènediamines, les para-aminophénols, les ortho-aminophénols, les bases hétérocycliques et leurs sels d'addition.
Préférentiellement, la ou les bases d'oxydation de l'invention sont choisies parmi les paraphénylènediamines et les bases hétérocycliques. Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino-N,N-diéthyl-3-méthylaniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino-2-chloroaniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2 hydroxyméthyl paraphénylènediamine, la 2-méthoxyméthyl paraphénylènediamine, la N,N-diméthyl-3-méthyl paraphénylènediamine, la N,N-(éthyl,-β-hydroxyéthyl) paraphénylènediamine, la N-(β,γ-dihydroxypropyl) paraphénylènediamine, la N-(4' aminophényl) paraphénylènediamine, la N-phényl paraphénylènediamine, la 2-β- hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.
Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, la 2-méthoxyméthyl paraphénylènediamine et leurs sels d'addition avec un acide sont particulièrement préférées.
Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl)-N,N'-bis-(4'-aminophényl)éthylènediamine, la N,N'-bis-(4-aminophényl) tétraméthylènediamine, la N,N'-bis-(β-hydroxyéthyl)-N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3' méthylphényl) éthylènediamine, le 1,8-bis-(2,5-diaminophénoxy)-3,6-dioxaoctane, et leurs sels d'addition.
Parmi les para-aminophénols, on peut citer à titre d'exemple, le para-amino phénol, le 4-amino-3-méthyl phénol, le 4-amino-3-fluoro phénol, le 4-amino-3-chlorophénol, le 4-amino-3-hydroxyméthyl phénol, le 4-amino-2-méthyl phénol, le 4-amino-2-hydroxyméthyl phénol, le 4-amino-2-méthoxyméthyl phénol, le 4-amino-2-aminométhyl phénol, le 4-amino 2-(β-hydroxyéthyl aminométhyl) phénol, le 4-amino-2-fluoro phénol, et leurs sels d'addition avec un acide.
Parmi les ortho-aminophénols, on peut citer à titre d'exemple, le 2-amino phénol, le 2-amino-5-méthyl phénol, le 2-amino-6-méthyl phénol, le 5-acétamido-2- amino phénol, et leurs sels d'addition.
Parmi les bases hétérocycliques, on peut en particulier les dérivés pyridiniques, les dérivés pyrimidiniques et les dérivés pyrazoliques.
Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 3,4-diamino pyridine, et leurs sels d'addition.
D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308.
A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine; l'acide 3-aminopyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-aminopyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-aminopyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-aminopyrazolo[1,5-a]pyridine-7-ol ; le 2-β-hydroxyéthoxy-3-aminopyrazolo[1,5-a]pyridine; le 2-(4-diméthylpypérazinium-1-yl)-3-aminopyrazolo[1,5-a]pyridine; ainsi que leurs sels d'addition.
Plus particulièrement les bases d'oxydation selon l'invention sont choisies parmi les 3-aminopyrazolo-[1,5 a]-pyridines de préférence substituées en position 2 par :

a) un groupe (di)($C_1$-$C_6$)(alkyl)amino les groupes alkyles pouvant être substitués par un ou plusieurs groupes hydroxy, amino, ou imidazolium ;

b) un groupe hétérocycloalkyle comprenant de 5 à 7 chaînons, et de 1 à 3 hétéroatomes, cationique ou non,

éventuellement substitué par un ou plusieurs groupes ($C_1$-$C_6$)alkyle tel que di(Ci-$C_4$)alkylpipérazinium ;

c) un groupe ($C_1$-$C_6$)alkoxy éventuellement substitué par un ou plusieurs groupes hydroxy tel que β-hydroxyalkoxy ainsi que leurs sels d'addition.

Parmi les dérivés pyrimidiniques, on peut citer les composés décrits par exemple dans les brevets DE 2359399 ; JP 88 169571 ; JP 05-63124 ; EP 0770375 ou demande de brevet WO 96/15765 comme la 2,4,5,6-tétra-aminopyrimidine, la 4 hydroxy 2,5,6-triaminopyrimidine, la 2 hydroxy 4,5,6-triaminopyrimidine, la 2,4 dihydroxy 5,6-diaminopyrimidine, la 2,5,6 triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5 diamino 1-méthyl pyrazole, le 4,5-diamino 1-(β-hydroxyéthyl) pyrazole, le 3,4-diamino pyrazole, le 4,5-diamino-1- (4'-chlorobenzyl) pyrazole, le 4,5-diamino-1,3-diméthyl pyrazole, le 4,5-diamino-3-méthyl-1-phényl pyrazole, le 4,5-diamino-1-méthyl-3-phényl pyrazole, le 4-amino-1,3-diméthyl-5-hydrazino pyrazole, le 1-benzyl-4,5-diamino-3-méthyl pyrazole, le 4,5-diamino-3-tert-butyl-1-méthyl pyrazole, le 4,5-diamino-1-tert-butyl 3-méthyl pyrazole, le 4,5-diamino-1-(β-hydroxyéthyl)-3-méthyl pyrazole, le 4,5-diamino-1-éthyl-3-méthyl pyrazole, le 4,5-diamino-1-éthyl-3-(4'-méthoxyphényl) pyrazole, le 4,5-diamino-1-éthyl 3-hydroxyméthyl pyrazole, le 4,5-diamino-3-hydroxyméthyl-1-méthyl pyrazole, le 4,5-diamino-3-hydroxyméthyl-1-isopropyl pyrazole, le 4,5-diamino-3-méthyl-1-isopropyl pyrazole, le 4-amino-5-(2'-aminoéthyl)amino-1,3-diméthyl pyrazole, le 3,4,5-triamino pyrazole, le 1-méthyl-3,4,5-triamino pyrazole, le 3,5-diamino-1-méthyl-4-méthylamino pyrazole, le 3,5-diamino-4-(β-hydroxyéthyl)amino-1-méthyl pyrazole, et leurs sels d'addition. De préférence les bases d'oxydation hétérocycliques de l'invention sont choisies parmi les 4,5-diaminopyrazoles telle que le 4,5-diamino-1-(β-hydroxyéthyl) pyrazole. On peut aussi utiliser le 4-5-diamino-1-(β-méthoxyéthyl)pyrazol e.

De préférence, on utilisera un 4,5-diaminopyrazole et encore plus préférentiellement le 4,5-diamino-1-(β-hydroxyéthyl)-pyrazole et/ou l'un de ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,SH-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,SH-pyrazolo[1,2-a]pyrazol-1-one et/ou un de leurs sels.

La ou les bases d'oxydation utilisées dans le cadre de l'invention sont en général présentes en quantité allant de 0,001 à 10 % en poids environ du poids total de la composition colorante, de préférence allant de 0,005 à 5 %.

Les coupleurs additionnels conventionnellement utilisés pour la teinture de fibres kératiniques sont de préférence choisis parmi les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro-1,3-dihydroxy benzène, le 1-hydroxy-3-aminobenzène, le 2-méthyl-5-aminophénol, le 3-amino-2-chloro-6-méthyl phénol, le 2-méthyl-5-hydroxyéthylaminophénol, le 2,4-diamino-1-(β-hydroxyéthyloxy) benzène, le 2-amino-4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le thymol, le 1-β-hydroxyéthylamino-3,4-méthylènedioxybenzène, l'a-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H-3-méthyl pyrazole-5-one, la 1-phényl-3-méthyl pyrazole 5-one, le 2,6-diméthyl pyrazolo-[1,5-b]-1,2,4-triazole, le 2,6-diméthyl-[3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Dans le cadre de la présente invention, lorsqu'ils sont présents, le ou les coupleurs sont généralement présents

en quantité totale allant de 0,001 à 10 % en poids environ du poids total de la composition colorante, de préférence allant de 0,005 à 5 % en poids par rapport au poids total de la composition colorante.

De préférence, la teneur totale en colorants d'oxydation dans la composition selon l'invention est comprise entre 0,001 à 20% en poids ; de préférence entre 0,001% et 10% en poids, de préférence entre 0,01% et 5% en poids, par rapport au poids de la composition.

Selon un mode de réalisation particulier, la composition comprend au moins une base d'oxydation et au moins un coupleur.

***Gommes de scléroglucane***

Selon l'invention, la composition (A) comprend une ou plusieurs gommes de scléroglucane en une teneur totale supérieure ou égale à 0,5% en poids par rapport au poids de la composition.

Les gommes de scléroglucane sont des polysaccharides d'origine microbienne produits par un champignon de type Sclerotium, en particulier Sclerotium rolfsii. Ce sont des polysaccharides constitués uniquement de motifs glucose.

Les gommes de scléroglucane peuvent être modifiées ou non. De préférence les gommes de scléroglucane utilisées dans la présente invention sont non modifiées.

Des exemples de gommes de scléroglucane utilisables dans la présente invention sont, de manière non limitative, les produits vendus sous la dénomination ACTIGUM CS, en particulier ACTIGUM CS 11, par la société SANOFI BIO INDUSTRIES et sous la dénomination AMIGUM ou AMIGEL par la société ALBAN MULLER INTERNATIONAL.

D'autres gommes de scléroglucane, telles que celle traitée au glyoxal décrite dans la demande de brevet français n° 2 633 940, peuvent également être utilisées.

La ou les gommes de scléroglucane utilisables selon l'invention représentent de préférence une teneur totale allant de 0,5 à 10 % en poids, plus préférentiellement de 0,5 à 5 % en poids, et encore plus préférentiellement de 0,5 à 3 % en poids, mieux de 0.5% à 2% en poids et encore plus préférentiellement de 0,7 à 1,5 % en poids, par rapport au poids total de la composition (A).

Selon un mode de réalisation de l'invention, la composition oxydante (B) comprend une ou plusieurs gommes de scléroglucane, de préférence en une teneur totale supérieure ou égale à 0,5% en poids par rapport au poids de la composition.

De préférence selon ce mode de réalisation, la ou les gommes de scléroglucane utilisables selon l'invention représentent de préférence de 0,5 à 10 % en poids, plus préférentiellement de 0,5 à 5 % en poids, et encore plus préférentiellement de 0,5 à 3 % en poids, encore plus préférentiellement et encore plus préférentiellement de 0,5 à 2 %, voire de 0,7 à 2% en poids, par rapport au poids total de la composition oxydante (B).

***Agents Alcalins***

La composition selon l'invention comprend un ou plusieurs agent(s) alcalin(s) minéral(aux) et un ou plusieurs agent(s) alcalin(s) organique(s) choisi(s) parmi les alcanolamine(s).

Les « agents alcalins » peuvent indifféremment être appelés des « agents alcalinisants ».

Par « agent alcalin minéral » (également appelé agent alcalin inorganique), on entend un agent alcalin qui n'est pas organique.

Le ou les agent(s) alcalin(s) minéraux sont de préférence choisis parmi l'ammoniaque, encore appelé hydroxyde d'ammonium, (ou agents précurseurs d'ammoniac comme les sels d'ammonium, par exemple les halogénures d'ammonium et en particulier le chlorure d'ammonium), les silicates, les phosphates, les carbonates ou bicarbonates de métaux alcalins ou alcalino-terreux, tels que métasilicates de métaux alcalins ou alcalino-terreux, les carbonates ou bicarbonates de sodium ou de potassium, les hydroxydes de sodium ou de potassium, ou leurs mélanges.

De façon préférée, le ou les agents alcalins minéraux sont choisis parmi l'ammoniaque (ou agents précurseurs d'ammoniac comme les sels d'ammonium, par exemple les halogénures d'ammonium et en particulier le chlorure d'ammonium) et/ou les métasilicates de métaux alcalins ou alcalino-terreux.

De façon préférée le ou les agents alcalins minéraux, est/sont présente(s) en une teneur totale allant de 0,1 à 10% en poids, plus préférentiellement de 0,5 à 8% en poids, mieux de 1 à 7% en poids par rapport au poids total de la composition.

Lorsque la composition comprend de l'ammoniaque (hydroxyde d'ammonium), sa teneur va de préférence de 0,1 à 10% en poids, plus préférentiellement de 0,5 à 8% en poids, mieux de 1 à 6% en poids par rapport au poids total de la composition.

La composition selon l'invention comprend également un ou plusieurs agents alcalins organiques choisis parmi les alcanolamines.

Par alcanolamine on entend une amine organique comprenant une fonction amine primaire, secondaire ou

tertiaire, et un ou plusieurs groupements alkyle, linéaires ou ramifiés, en $C_1$ à $C_8$ porteurs d'un ou plusieurs radicaux hydroxy.

De préférence les amines organiques choisies parmi les alcanolamines telles que les mono-, di- ou tri-alcanolamines, comprenant un à trois radicaux hydroxyalkyle, identiques ou non, en $C_1$ à $C_4$.

De préférence les alcanolamines sont de préférence choisies parmi la monoéthanolamine (MEA), la diéthanolamine, la triéthanolamine, la monoisopropanolamine, la diisopropanolamine, la N,N-diméthyléthanolamine, le 2-amino-2-méthyl-1-propanol, la triisopropanol-amine, le 2-amino-2-méthyl-1,3-propanediol, le 3-amino-1,2-propanediol, le 3-diméthylamino-1,2-propanediol, le tris-hydroxyméthylamino-méthane, et leurs mélanges, de préférence la monoéthanolamine (MEA).

De façon préférée la ou les alcanolamines est/sont présente(s) en une teneur totale allant de préférence de 0,5 à 10% en poids, plus préférentiellement de 1 à 9% en poids, mieux de 2 à 8% en poids par rapport au poids total de la composition.

De façon préférée, le ratio pondéral de la teneur totale en agents alcalins minéraux par apport à la teneur totale en alcanolamines allant de 0.05 à 10, plus préférentiellement de 0.1 à 5 et mieux de 0.15 à 3.

De façon particulièrement préférée, la composition selon l'invention comprend de l'ammoniaque (hydroxyde d'ammonium) et une ou plusieurs alcanolamine(s), de préférence la monoéthanolamine.

**Agents alcalins additionnels**

La composition selon l'invention peut en outre comprendre un ou plusieurs agents alcalins additionnels, différents des agents alcalins minéraux et des alcanolamines.

De préférence le ou les agents alcalins additionnels peuvent être choisis parmi les agents alcalins organiques différents des alcanolamines.

A titre d'exemple, on peut citer les acides aminés.

A titre d'acides aminés utilisables dans la composition selon la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les agents alcalins additionnels sont choisis parmi les acides aminés basiques, notamment comprenant une fonction amine supplémentaire éventuellement incluse dans un cycle ou dans une fonction uréido.

De préférence, le ou les agents alcalins additionnels sont choisis parmi les acides aminés, de préférence basiques, de préférence l'arginine.

De façon préférée lorsque que la composition selon l'invention contient un ou plusieurs agents alcalins additionnels différent(s) des agents alcalins minéraux et des alcanolamines, ils sont présents en une teneur totale allant de 0,5 à 10% en poids, plus préférentiellement de 1 à 8% en poids, mieux de 2 à 7% en poids par rapport au poids total de la composition.

**Polymère Associatif**

La composition selon l'invention peut également comprendre un ou plusieurs polymères associatifs. Les polymères associatifs selon l'invention sont des polymères comprenant au moins une chaîne grasse en $C_8$ - $C_{30}$ et dont les molécules sont capables, dans le milieu de formulation, de s'associer entre elles ou avec des molécules d'autres composés.

De préférence, la chaîne grasse comporte de 10 à 30 atomes de carbone.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

Les polymères associatifs pouvant être utilisés dans la composition selon l'invention peuvent être choisis parmi les polymères associatifs non ioniques, anioniques, cationiques et amphotères, et leurs mélanges.

Selon un mode de réalisation de l'invention, le ou les polymère(s) associatifs(s) est/sont choisi(s) parmi les polymères associatifs non ioniques.

Les polymères associatifs de type non ionique sont choisis de préférence parmi :

- (1) les celluloses modifiées par des groupements comportant au moins une chaîne grasse ;
  De préférence parmi :

- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, , de préférence comme la cetylhydroxyéthyl cellulose commercialisée notamment sous la référence NATROSOL PLUS GRADE 330 CS (alkyles en $C_{16}$) vendu par la société Ashland, ou le produit Polysurf 67CS vendu par la société Ashland,
- les hydroxyéthylcelluloses modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL
- et leurs mélanges.

- (2) les hydroxypropylguars modifiés par des groupements comportant au moins une chaîne grasse tel que le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHODIA.

- (3) les copolymères de vinyl pyrrolidone et de monomères hydrophobes à chaîne grasse ;
on peut citer à titre d'exemple :

  - les produits ANTARON V216 ou GANEX V216 (copolymère vinylpyrrolidone / hexadécène) vendu par la société I.S.P.
  - les produits ANTARON V220 ou GANEX V220 (copolymère vinylpyrrolidone / eicosène) vendu par la société I.S.P.

- (4) les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

- (7) les polymères à squelette aminoplaste éther possédant au moins une chaîne grasse, tels que les composés PURE THIX proposés par la société SUD-CHEMIE.

- (8) et leurs mélanges.

De façon particulièrement préférée, le ou les polymères associatifs sont non ioniques, et de préférence choisis parmi les celluloses modifiées par des groupements comportant au moins une chaîne grasse.
De préférence, le ou les polymères associatifs non ioniques sont choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et les hydroxyéthylcelluloses modifiées par des groupes polyalkylène glycol éther d'alkyl phénol, et leurs mélanges, de préférence la cétylhydroxyethylcellulose.

***Polymères cationiques***

Selon un mode de réalisation avantageux de l'invention, la composition comprend un ou plusieurs polymères cationiques,
**[0093]** Comme polymères cationiques utilisables dans les compositions selon l'invention, on peut citer en particulier :

(1) les cyclopolymères d'alkyl diallyl amine ou de dialkyl diallyl ammonium tels que les homopolymères ou copolymères comportant comme constituant principal de la chaîne des motifs répondant aux formules (I) ou (II) :

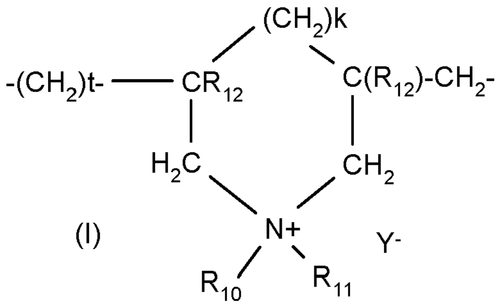

(I)

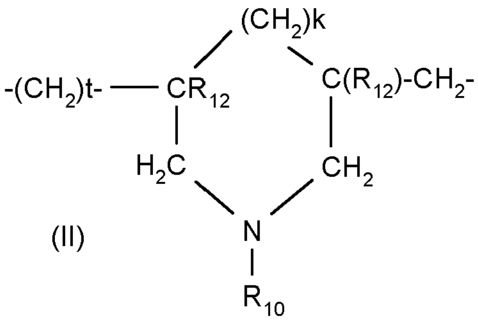

(II)

dans lesquelles

- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle en C1-C6, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11, indépendamment l'un de l'autre, désignent de préférence un groupement alkyle en C1-C4;
- $Y^-$ est un anion tel que bromure, chlorure, acétate, borate, citrate, tartrate, bisulfate, bisulfite, sulfate, phosphate.

On peut citer plus particulièrement l'homopolymère de sels (par exemple chlorure) de diméthyldiallylammonium par exemple vendu sous la dénomination "MERQUAT 100" par la société NALCO, De préférence, les polymères de la famille (1) sont choisis parmi les homopolymères de dialkyl diallyl ammonium.
(2) les polymères de diammonium quaternaire comprenant des motifs récurrents de formule :

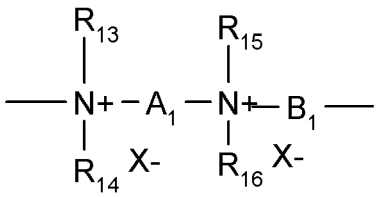

(III)

dans laquelle :

- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- $X^-$ désigne un anion dérivé d'un acide minéral ou organique;

étant entendu que A1, R13 et R15 peuvent former avec les deux atomes d'azote auxquels ils sont rattachés un cycle pipérazinique ;
en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désignant :

a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine
c) un reste de diamine bis-primaire de formule -NH-Y-NH- où Y désigne un radical hydrocarboné linéaire ou ramifié, ou bien le radical divalent -CH2-CH2-S-S-CH2-CH2- ;
d) un groupement uréylène de formule -NH-CO-NH- . De préférence, $X^-$ est un anion tel que le chlorure ou le bromure. Ces polymères ont une masse molaire moyenne en nombre (Mn) généralement comprise entre 1000 et 100000.

On peut citer plus particulièrement les polymères cationiques qui sont constitués de motifs récurrents répondant à la formule :

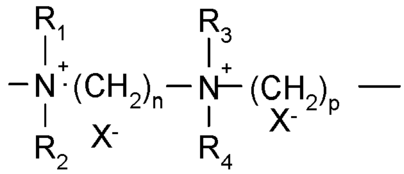
(IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique.
Un composé de formule (IV) particulièrement préféré est celui pour lequel R1, R2, R3 et R4 représentent un radical méthyle, n=3, p=6 et X = Cl, dénommé Hexadimethrine chloride selon la nomenclature INCI (CTFA).
De préférence, le ou les polymère(s) cationique(s) est/sont choisi(s) parmi les homopolymères de dialkyl diallyl ammonium, en particulier les homopolymères de sels de diméthyldiallylammonium, les polymères constitués de motifs récurrents répondant à la formule (IV) ci-dessus, en particulier le poly(dimethyliminio)-1,3-propanediyl(dimethyliminio)-1,6-hexanediyldichloride, dont le nom INCI est hexadimethrine chloride ; et leurs mélanges.
Lorsqu'ils sont présents, la teneur totale en polymères cationiques dans la composition selon la présente invention peut varier de 0,01 à 10% en poids par rapport au poids de la composition, de préférence de 0,1 à 7 %, par rapport au poids de la composition, encore plus avantageusement de 0,5à 5 % en poids, mieux de 0,5 à 3 % en poids par rapport au poids de la composition.

***Acides carboxyliques***

La composition colorante (A) selon l'invention peut avantageusement comprendre un ou plusieurs acides carboxyliques, et/ou leurs sels d'addition et/ou leurs solvates, ledit ou lesdits acides carboxyliques étant des composés aliphatiques, comprenant de 2 à 10 atomes de carbone et comprenant de préférence au moins deux groupements carboxyliques.
De façon préférés, ils sont choisis parmi les acides dicarboxyliques et/ou tricarboxyliques aliphatiques comprenant de 2 à 10 atomes de carbone, de préférence de 2 à 8 atomes de carbone, mieux de 2 à 6 atomes de carbone.
De préférence, les acides carboxyliques peuvent être choisis parmi l'acide oxalique, l'acide malonique, l'acide malique, l'acide glutarique, l'acide citraconique, l'acide citrique, l'acide maléique, l'acide succinique, l'acide adipique, l'acide tartrique, l'acide fumarique, et leurs mélanges.
De façon préférée, le ou les acides carboxyliques comprennent au moins deux groupes carboxyliques et sont choisis parmi l'acide malonique, l'acide citrique, l'acide maléique, l'acide glutarique, l'acide succinique et leurs mélanges ; de préférence choisis parmi l'acide malonique, l'acide citrique, l'acide maléique,et leurs mélanges.
De manière plus particulièrement préférée, l'acide carboxylique est l'acide citrique.
La teneur totale en acide(s) carboxylique(s) et/ou leurs sels d'addition et/ou leurs solvates varie de préférence de 0.1% à 20% en poids, par rapport au poids total de la composition (A).
De préférence, la teneur totale en acide(s) carboxylique(s) varie de 0,1 à 20, préférentiellement de 0.5 % à 10% en poids, mieux de 1% à 7% en poids, par rapport au poids total de la composition, mieux encore de 2 à 5 % en poids par rapport au poids total de la composition (A).

***Tensioactifs***

De préférence, la composition selon la présente invention comprend également un ou plusieurs agents tensioactifs, ceux-ci peuvent être choisis parmi les agents tensioactifs anioniques, les agents tensioactifs amphotères ou zwittérioniques, les agents tensioactifs non-ioniques et les agents tensioactifs cationiques, et leurs mélanges, de préférence parmi les agents tensioactifs non ioniques, les agents tensioactifs cationiques et leurs mélanges.
On entend par « agent tensioactif anionique », un tensioactif ne comportant à titre de groupements ioniques ou ionisables que des groupements anioniques. Ces groupements anioniques sont choisis de préférence parmi les groupements $CO_2H$, $CO_2^-$, $SO_3H$, $SO_3^-$, $OSO_3H$, $OSO_3^-$, $H_2PO_3$, $HPO_3^-$, $PO_3^{2-}$, $H_2PO_2$, $HPO_2^-$, $PO_2^{2-}$, POH et $PO^-$.
Selon un mode de réalisation, la composition selon l'invention comprend un ou plusieurs agents tensioactifs non ionique(s).
Les tensioactifs non ioniques utilisables selon l'invention peuvent être choisis parmi :

- les alcools, les alpha-diols et les alkyl($C_1$-$C_{20}$)phénols, ces composés étant polyéthoxylés et/ou polypropoxylés et/ou polyglycérolés, le nombre de groupements oxyde d'éthylène et/ou oxyde de propylène pouvant aller de 1 à 100, et le nombre de groupements glycérol pouvant aller de 2 à 30; ou bien ces composés comprenant au moins une chaîne grasse comportant de 8 à 40 atomes de carbone, notamment de 16 à 30 atomes de carbone ; en particulier les alcools comprenant au moins une chaîne alkyle en $C_8$ à $C_{40}$, saturés ou non, linéaires ou ramifiés, oxyéthylénés comprenant de 1 à 100 moles d'oxyde d'éthylène, de préférence de 2 à 50, plus particulièrement de 2 à 40 moles d'oxyde d'éthylène et comportant une ou deux chaînes grasses;
- les condensats d'oxyde d'éthylène et d'oxyde de propylène sur des alcools gras;
- les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4;
- les esters d'acides gras du sorbitane éthoxylés ayant de préférence de 2 à 40 motifs d'oxyde d'éthylène ;
- les esters d'acides gras du saccharose ;
- les esters d'acides gras polyoxyalkylénés, de préférence polyoxyéthylénés ayant de 2 à 150 moles d'oxyde d'éthylène dont les huiles végétales oxyéthylénées ;
- les dérivés de N-(alkyl en $C_6$-$C_{24}$)glucamine,
- les oxydes d'amines tels que les oxydes d'(alkyl en $C_{10}$-$C_{14}$)amines ou les oxydes de N-(acyl en $C_{10}$-$C_{14}$)-aminopropylmorpholine ;
- et leurs mélanges.

Parmi les produits commerciaux, on peut citer les produits vendus par la société COGNIS sous les dénominations PLANTAREN® (600 CS/U, 1200 et 2000) ou PLANTACARE® (818, 1200 et 2000); les produits vendus par la société SEPPIC sous les dénominations ORAMIX CG 110 et ORAMIX® NS 10; les produits vendus par la société BASF sous la dénomination LUTENSOL GD 70 ou encore les produits vendus par la société CHEM Y sous la dénomination AG10 LK.
De préférence, on utilise les alkyl $C_8/C_{16}$-(poly)glycoside 1,4, notamment en solution aqueuse à 53%, tels que ceux commercialisés par COGNIS sous la référence PLANTACARE® 818 UP.
Préférentiellement, les tensioactifs non ioniques sont choisis parmi :

- les alcools gras oxyéthylénés, comportant au moins une chaîne alkyle en $C_8$ à $C_{40}$, notamment en $C_8$-$C_{20}$, encore mieux en $C_{10}$-$C_{18}$ , saturée ou non, linéaire ou ramifiée, et comprenant de 1 à 100 moles d'oxyde d'éthylène, de préférence de 2 à 50, plus particulièrement de 2 à 40 moles, voire de 3 à 20 moles d'oxyde d'éthylène;, et
- les (alkyl $C_6$-$C_{24}$)(poly)glycosides, et plus particulièrement les (alkyl $C_8$-$C_{18}$)(poly)glycosides ;
- et leurs mélanges ;

et plus préférentiellement encore parmi les (alkyl $C_6$-$C_{24}$)(poly)glycosides, préférentiellement les (alkyl $C_8$-$C_{18}$)(poly)glycosides.
Selon un mode de réalisation préféré de l'invention, la composition selon l'invention comprend un ou plusieurs tensioactifs non ioniques, de préférence choisis parmi les alkyl(poly)glycosides. De préférence la composition selon l'invention comprend un ou plusieurs tensioactifs choisi parmi les (alkyl $C_6$-$C_{24}$)(poly)glycosides, plus préférentiellement parmi les (alkyl $C_8$-$C_{18}$)(poly)glycosides, de préférence parmi les alkyl $C_8/C_{16}$-(poly)glucosides, de préférence de type 1,4, et de préférence choisi parmi les décylglucosides et/ou les caprylyl/capryl glucosides et/ou cocoglucosides.
Selon un premier mode de réalisation, le ou les agents tensioactifs sont non ioniques, de préférence choisis parmi les (alkyl en $C_6$-$C_{24}$)polyglycosides.
Selon un mode de réalisation préféré, la composition selon l'invention comprend au moins un ou plusieurs agents tensioactifs cationiques. De préférence, le ou les tensioactifs sont choisis parmi les sels d'amines grasses

primaires, secondaires ou tertiaires, éventuellement polyoxyalkylénées, les sels d'ammonium quaternaire, et leurs mélanges.

De préférence, le ou les agents tensioactifs cationiques, sont choisis parmi les sels de cétyltriméthylammonium, de béhényltriméthylammonium, de dipalmitoyléthylhydroxy éthylméthylammonium, et leurs mélanges, et plus particulièrement le chlorure de béhényltriméthylammonium, le chlorure de cétyltriméthylammonium, le méthosulfate de dipalmitoyléthylhydroxy éthylammonium, et leurs mélanges.

La composition comprend, de préférence un ou plusieurs agents tensioactifs en une teneur totale allant de 0,01 à 20 % en poids, plus préférentiellement de 0,05 à 10 % en poids, mieux de 0,1 à 5 % en poids, par rapport au poids total de la composition.

La composition comprend, de préférence un ou plusieurs agents tensioactifs non ioniques en une teneur totale allant de 0,01 à 10 % en poids, plus préférentiellement de 0,05 à 5 % en poids, mieux de 0,1 à 3 % en poids, par rapport au poids total de la composition

La composition comprend, de préférence un ou plusieurs agents tensioactifs cationiques en une teneur totale allant de 0,01 à 10 % en poids, plus préférentiellement de 0,05 à 5 % en poids, mieux de 0,1 à3 % en poids, par rapport au poids total de la composition.

De manière préférée, le ou les agents tensioactifs sont choisis parmi les tensioactifs cationiques ou non ioniques, et leurs mélanges, préférentiellement cationiques. De préférence la composition selon l'invention comprend au moins un ou plusieurs tensioactifs cationiques et un ou plusieurs tensioactifs non ioniques.

***Milieu***

Le milieu cosmétiquement acceptable approprié pour la coloration des fibres kératiniques, appelé aussi « support » de coloration, comprend généralement de l'eau ou un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant de 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; le glycérol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants usuels décrits ci-dessus, s'ils sont présents, représentent habituellement de 1 à 40% en poids, plus préférentiellement de 5 à 30% en poids, par rapport au poids total de la composition.

Les compositions mises en oeuvre selon l'invention comprennent généralement de l'eau ou un mélange d'eau et d'un ou plusieurs solvants organiques ou un mélange de solvants organiques.

La composition selon l'invention comprend de préférence de l'eau.

De préférence la teneur en eau varie de 5 à 95% en poids, plus préférentiellement de 10 à 90 % en poids, mieux de 20 à 80 % en poids, par rapport au poids total de la composition.

***pH du milieu***

Le pH de la composition selon l'invention varie généralement de 1 à 12 De façon préférée, le pH de la composition (A) selon l'invention est basique.

Par « pH basique » au sens de la présente invention, on entend un pH supérieuà 7.

De préférence, le pH de la composition (A) selon l'invention est supérieur à 8, et particulièrement va de 8,5 à 12.

De préférence le pH de la composition est compris entre 9 et 12.

*Ajusteur de pH*

Le milieu cosmétiquement acceptable peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, (ortho)phosphorique, l'acide borique, l'acide nitrique, l'acide sulfurique ou les acides organiques comme par exemple les composés comprenant au moins une fonction acide sulfonique, une fonction acide phosphonique ou une fonction acide phosphorique, ou les composés à fonction acide carboxylique tels que ceux décrits précédemment.

***Autres additifs***

La composition selon l'invention peut également renfermer divers additifs utilisés classiquement dans les compositions pour la coloration des cheveux, tels que des agents épaississants minéraux, et en particulier des charges telles que des argiles, le talc ; des agents épaississants organiques différents des gommes de scléroglucane des agents antioxydants ; des agents de pénétration ; des agents séquestrants ; des parfums ; des agents dispersants ; des agents filmogènes ; des céramides ; des agents conservateurs ; des agents opacifiants, des corps gras et/ou des colorants directs additionnels.

Les additifs ci-dessus sont en général présents en quantité comprise pour chacun d'eux entre 0,01 et 40 % en poids par rapport au poids de la composition, de préférence entre 0,05 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

**Procédé de coloration**

Un autre objet de l'invention est un procédé de coloration mettant en oeuvre une composition de coloration (A) telle que décrite précédemment, avec une composition oxydante (B) comprenant un ou plusieurs agents oxydants chimiques.

En particulier, L'invention vise également un procédé de coloration des fibres kératiniques, et en particulier des fibres kératiniques humaines telles que les cheveux, mettant en oeuvre l'application sur les fibres d'une composition colorante (A) telle que définie précédemment, et d'une composition oxydante (B) comprenant au moins un agent oxydant chimique, de préférence choisi parmi le peroxyde d'hydrogène et/ou un ou plusieurs système(s) générateur(s) de peroxyde d'hydrogène, de préférence parmi le peroxyde d'hydrogène, la composition oxydante (B) étant mélangée avec la composition colorante (A) juste avant l'emploi (ie application sur lesdites fibres) (extemporanément) ou au moment de l'emploi, ou bien les compositions colorantes et oxydantes étant appliquées séquentiellement sans rinçage intermédiaire.

**Agent oxydant :**

La composition oxydante (B) mise en oeuvre avec la composition colorante (A) selon l'invention contient un ou plusieurs agent(s) oxydant(s) chimique(s), de préférence choisi(s) parmi le peroxyde d'hydrogène et/ou un ou plusieurs système(s) générateur(s) de peroxyde d'hydrogène.

Par « agent oxydant chimique » on entend un agent oxydant différent de l'oxygène de l'air.

De préférence, le ou les agents oxydants chimiques sont choisis parmi le peroxyde d'hydrogène, les sels peroxygénés comme par exemple les persulfates, les perborates, les peracides et leurs précurseurs, les percarbonates de métaux alcalins ou alcalino-terreux tel que le peroxyde de carbonate de sodium ou appelé percarbonate de sodium et les peracides et leurs précurseurs ; les bromates ou ferricyanures de métaux alcalins, les agents oxydants chimiques solides générateurs de peroxyde d'hydrogène tels que le peroxyde d'urée et les complexes polymériques pouvant libérer du peroxyde d'hydrogène notamment ceux comprenant un monomère hétérocyclique vinylique tels que les complexes polyvinylpyrrolidone/$H_2O_2$ en particulier se présentant sous forme de poudres ; les oxydases produisant du peroxyde d'hydrogène en présence d'un substrat adéquat (par exemple le glucose dans le cas de glucose oxydase ou l'acide urique avec l'uricase).

De manière préférée, le ou les agents oxydants chimiques sont choisis parmi le peroxyde d'hydrogène, le peroxyde d'urée, les bromates ou ferricyanures de métaux alcalins, les sels peroxygénés, et les mélanges de ces composés,

De manière particulièrement préférée, l'agent oxydant chimique est du peroxyde d'hydrogène.

De manière préférée, le ou les agents oxydants chimiques représentent de 0,05 à 40 % en poids, de préférence de 0,5 à 30 % en poids, plus préférentiellement de 1 à 20 % en poids, et mieux de 1,5 à 15 % en poids par rapport au poids total de la composition oxydante (B).

De préférence, la composition oxydante (B)selon l'invention ne contient pas de sels peroxygénés.

Comme indiqué précédemment, selon un mode de réalisation de l'invention, la composition oxydante (B) comprend une ou plusieurs gommes de scléroglucane, de préférence en une teneur totale supérieure ou égale à 0,5% en poids par rapport au poids de la composition.

De préférence selon ce mode de réalisation, la ou les gommes de scléroglucane utilisables selon l'invention représentent de préférence de 0,5 à 10 % en poids, plus préférentiellement de 0,5 à 5 % en poids, et encore plus

préférentiellement de 0,5 à 3 % en poids, encore plus préférentiellement et encore plus préférentiellement de0,7 à 2% en poids, par rapport au poids total de la composition oxydante (B).

La composition oxydante (B) peut également renfermer divers composés additionnels ou divers adjuvants utilisés classiquement dans les compositions pour la coloration des cheveux notamment tels que définis précédemment.

La composition oxydante (B) est généralement une composition aqueuse. Par composition aqueuse, on entend au sens de l'invention une composition comprenant plus de 20 % en poids d'eau, de préférence plus de 30 % en poids d'eau, et de manière encore plus avantageuse plus de 40 % en poids d'eau.

De préférence, la composition oxydante (B) comprend usuellement de l'eau qui représentent généralement de 10 à 98 % en poids, de préférence de 20 à 96 % en poids, de préférence de 50 à 95 % en poids, par rapport au poids total de la composition.

Cette composition oxydante (B) peut également comprendre un ou plusieurs solvants organiques hydrosolubles tels que décrits précédemment. Elle peut aussi comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition (B), est inférieur à 7.

Le pH de la composition (B) de l'invention est avantageusement compris entre 1 et 7, de préférence entre 1 et 4, et plus préférentiellement de 1,5 à 3,5.

Enfin, la composition oxydante (B) se présente sous diverses formes, comme par exemple une solution, une émulsion ou un gel.

***PROCEDE DE COLORATION***

Le procédé de l'invention peut être mis en oeuvre en appliquant la composition colorante (A) telle que définie précédemment et la composition oxydante (B) successivement et sans rinçage intermédiaire, l'ordre étant indifférent.

Selon une autre variante préférée, on applique sur les matières kératiniques, sèches ou humides, une composition prête à l'emploi obtenue par mélange extemporané, au moment de l'emploi, de la composition colorante (A) telle que définie précédemment et de la composition oxydante (B). Selon ce mode de réalisation, de préférence le rapport pondéral R des quantités de (A) / (B) varie de 0,1 à 10 de préférence de 0,2 à 2, mieux de 0,3 à 1.

En outre, indépendamment de la variante mise en oeuvre, l'application de la composition prête à l'emploi sur les matières kératiniques (résultant soit du mélange extemporané des compositions colorantes (A) et oxydantes (B) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les matières kératiniques sont éventuellement rincées à l'eau, subissent éventuellement un lavage suivi d'un rinçage à l'eau, avant d'être séchées ou laissées à sécher.

De préférences les fibres kératiniques sont des fibres kératiniques humaines, de préférence des cheveux humains.

L'invention a également pour objet une composition de coloration prête à l'emploi des fibres kératiniques, en particulier des fibres kératiniques humaines, telles que les cheveux, obtenue par mélange extemporané, au moment de l'emploi d'une composition (A) comprenant:

- un ou plusieurs colorant(s) d'oxydation ;
- une ou plusieurs gommes de scléroglucane, en une teneur totale supérieure ou égale à 0,5% en poids, par rapport au poids de la composition ;
- un ou plusieurs agent(s) alcalin(s) minéral(aux) ; et
- un ou plusieurs agent(s) alcalin(s) organique(s) choisi(s) parmi les alcanolamine(s) ;
- et d'une composition (B) comprenant
- un ou plusieurs agent(s) oxydant(s) chimique(s).

Par « extemporané » on entend notamment moins de 30 minutes, de préférence moins de 15 minutes avant l'application sur les fibres kératiniques, de préférence moins de 5 minutes. En particulier, le mélange est appliqué immédiatement après sa préparation.

Selon un mode particulier de l'invention, le ou les agents oxydants chimiques représente(nt) de préférence une teneur totale variant de 0,1 à 20 % en poids, de préférence de 0,5 à 15% en poids, ou encore plus préférentiellement de 1 à 10% en poids, par rapport au poids total de la composition prête à l'emploi.

Enfin, l'invention concerne un dispositif à plusieurs compartiments comprenant dans un premier compartiment

une composition colorante (A) comme décrite auparavant, et dans un second, une composition oxydante (B) comprenant un ou plusieurs agents oxydants, ces compositions ayant été décrites auparavant.

En particulier, l'invention a également pour objet un dispositif (ou " kit ") à plusieurs compartiments permettant la mise en oeuvre de la composition de coloration des fibres kératiniques, de préférence comprenant au moins deux compartiments, un premier compartiment contenant la composition colorante (A) telle que définie précédemment, et le second compartiment contenant au moins une composition oxydante (B) comprenant au moins un agent oxydant chimique, de préférence choisi parmi le peroxyde d'hydrogène et/ou un ou plusieurs système(s) générateur(s) de peroxyde d'hydrogène, de préférence le peroxyde d'hydrogène, les compositions des compartiments étant destinées à être mélangées avant application, pour donner la formulation après mélange ; en particulier le kit peut être un dispositif aérosol.

Les exemples suivants servent à illustrer l'invention sans toutefois présenter un caractère limitatif.

En particulier, la coloration des fibres kératiniques obtenue dans ces exemples peut être avantageusement évaluée dans le système CIE L* a* b*, au moyen d'un Spectrocolorimètre Datacolor Spectraflash SF600X.

Dans ce système L* a* b*, les trois paramètres désignent respectivement l'intensité de la couleur (L*), a* indique l'axe de couleur vert/rouge et b* l'axe de couleur bleu/jaune. Plus la valeur de L* est élevée, plus la couleur est claire. Plus la valeur de a* est élevée, plus la couleur est rouge, plus la valeur de b* est élevée, plus la couleur est jaune.

La variation (ou l'importance) de la coloration entre les mèches de cheveux non traitées et les mèches de cheveux après traitement est définie par le paramètre DE* et est calculé selon l'équation suivante :

$$DE^* = \sqrt{(L^* - L_o^*)^2 + (a^* - a_o^*)^2 + (b^* - b_o^*)^2} \quad \text{(i)}$$

Dans cette équation, les paramètres L*, a* et b* représentent les valeurs mesurées sur les mèches de cheveux après la coloration et les paramètres $L_0$*, $a_0$* et $b_0$* représentent les valeurs mesurées sur les mèches de cheveux non traitées. Plus la valeur de DE* est grande, meilleure est la coloration des fibres kératiniques.

Dans le système CIE L*, a*, b*, la chromaticité est calculée selon l'équation suivante:

$$\dot{C}^* = \sqrt{a^{*2} + b^{*2}}$$

Plus la valeur de C* est élevée et plus la coloration est chromatique.

**<u>Exemple 1</u> :**

On a préparé les compositions suivantes à partir des ingrédients suivants dans les proportions suivantes indiquées en gramme de matière active :

| | Composition comparative C1 hors invention | Composition comparative C2 hors invention | Composition comparative C3 hors invention | Composition A1 (selon l'invention) |
|---|---|---|---|---|
| Ammonium Hydroxide | 2,47 | 2,47 | 2,47 | 2,47 |
| Ethanolamine | 4 | 4 | 4 | 4 |
| EDTA | 0,2 | 0,2 | 0,2 | 0,2 |
| Sodium Sulfite | 0,5 | 0,5 | 0,5 | 0,5 |
| Colorants d'oxydation | 1,401 | 1,401 | 1,401 | 1,401 |
| Fragance | qs | qs | qs | qs |
| Hexadimethrine Chloride | 0,3 | 0,3 | 0,3 | 0,3 |
| Polyquaternium -6 | 0,4 | 0,4 | 0,4 | 0,4 |

(suite)

| | Composition comparative C1 hors invention | Composition comparative C2 hors invention | Composition comparative C3 hors invention | Composition A1 (selon l'invention) |
|---|---|---|---|---|
| Cetyl hydroxyéthylcel lulose | 0,2 | 0,2 | 0,2 | 0,2 |
| Xanthan Gum | 1 | - | - | - |
| Algin | - | - | 1 | - |
| Sclerotium Gum | - | - | - | 1 |
| Hydroxypropyl cellulose | - | 1 | - | - |
| Eau | Qs 100 | Qs 100 | Qs 100 | Qs 100 |
| Glycérine | 10 | 10 | 10 | 10 |
| Cetrimonium Chloride | 0,25 | 0,25 | 0,25 | 0,25 |
| Caprylylcapryl Glucoside | 0,6 | 0,6 | 0,6 | 0,6 |
| Acide Ascorbique | 0,4 | 0,4 | 0,4 | 0,4 |

**Evaluation visuelle de la stabilité des compositions**

La stabilité des compositions colorantes a été évaluée en observant les compositions à T0 (immédiatement après préparation de la composition) puis après 2 mois de stockage à 45°C.

| | Composition C1 | Composition C2 | Composition C3 | Composition A1 |
|---|---|---|---|---|
| Observation à T0 à température ambiante (25°C) | Texture liquide Non homogène (déphasage) | Texture liquide Non homogène (déphasage) | Texture liquide Non homogène (déphasage) | Gel translucide Homogène (pas de déphasage) |
| Observation après 2 mois à 45°C | Texture liquide Non homogène (déphasage) | Texture liquide Non homogène (déphasage) | Texture liquide Non homogène (déphasage) | Gel translucide Homogène (pas de déphasage) |

On observe que la composition A1 selon l'invention est homogène et forme un gel translucide à T0. Après 2 mois à 45° la composition A1 selon l'invention est stable ; elle est homogène et translucide. Les compositions comparatives C1, C2 et C3 dans lesquelles la gomme de scléroglucane a été remplacée poids pour poids par un autre agent épaississant de type polysaccharide ne sont pas stables. En effet, elles ne sont pas homogènes, dès T0 on observe un déphasage de ces compositions.

**Exemple 2:**

On a préparé les compositions suivantes à partir des ingrédients suivants dans les proportions suivantes indiquées en gramme :

| | Composition A2 selon l'invention | Composition Comparative C4 hors invention |
|---|---|---|
| Ammonium Hydroxide | 2,47 | 2,47 |
| Ethanolamine | 4,47 | 4,47 |

(suite)

| | Composition A2 selon l'invention | Composition Comparative C4 hors invention |
|---|---|---|
| EDTA | 0,2 | 0,2 |
| Sodium Sulfite | 0,5 | 0,5 |
| TOLUENE-2,5-DIAMINE | 0,4 | 0,4 |
| 2-METHYL-5-HYDROXYETHYLAMINOPHENOL | 0,264 | 0,264 |
| 4-AMINO-2-HYDROXYTOLUENE | 1,304 | 1,304 |
| HYDROXYETHOXY AMINOPYRAZOLOPYRIDINE HCL | 1,76 | 1,76 |
| p-Aminophénol | 0,128 | 0,128 |
| Fragance | qs | qs |
| Cetyl Hydroxyéthylcellulose | 0,2 | 0,4 |
| **Sclerotium gum** | **0,6** | **0,4** |
| Eau | Qs 100 | qs 100 |
| Glycérine | 10 | 10 |
| Coco-bétaine | 0,15 | 0,15 |
| Caprylylcapryl Glucoside | 0,6 | 0,6 |
| Acide Ascorbique | 0,4 | 0,4 |

**Evaluation visuelle de la stabilité des compositions**

La stabilité des compositions colorantes a été évaluée en observant les compositions à T0 (immédiatement après préparation de la composition) puis après 2 mois de stockage à température ambiante (25°C), et après 2 mois de stockage à 45°C.

| | Composition A2 selon l'invention | Composition comparative C4 |
|---|---|---|
| Observation à T0 (immédiatement après préparation) | Homogène (Pas de Déphasage) Texture : Gel lisse | Homogène (Pas de Déphasage) Texture : Gel lisse |
| Observation après 2 mois à 25°C | Homogène (Pas de Déphasage) Texture : Gel lisse | Déphasage : Gel avec présence d'un relargage liquide |
| Observation après 2 mois à 45°C | Texture Gel lisse Homogène (pas de déphasage) | Déphasage : Gel avec présence d'un relargage liquide |

On observe que la composition A2 selon l'invention qui comprend une teneur en gomme de scléroglucane supérieure ou égale à 0,5% en poids par rapport au poids total de la composition est stable à température ambiante ainsi qu'à 45° pendant 2 mois, contrairement à la composition comparative C4 qui comprend une teneur en gomme de scléroglucane de 0,4% en poids, par rapport au poids de la composition. Les compostions A2 et C4 comprennent la même teneur totale en agent(s) épaississant(s) (0,8%). La composition comparative C4 n'est donc pas stable.

**Exemple 3**

On a préparé la composition suivante selon l'invention à partir des ingrédients suivants dans les proportions suivantes indiquées en gramme :

| **INCI US** | Composition A3 Selon l'invention |
|---|---|
| AMMONIUM HYDROXIDE | 2,47 |
| ETHANOLAMINE | 4,73 |
| EDTA | 0,2 |
| SODIUM SULFITE | 0,5 |
| TOLUENE-2,5 -DIAMINE | 0,16 |
| 4-AMINO-2-HYDROXYTOLUENE | 0,92 |
| 5-AMINO-6-CHLORO-o-CRESOL | 0,2 |
| 1-HYDROXYETHYL 4,5-DIAMINO PYRAZOLE SULFATE | 1,44 |
| p-AMINOPHENOL | 0,12 |
| FRAGRANCE | qs |
| CETYL HYDROXYETHYLCELLULOSE | 0,2 |
| SCLEROTIUM GUM | 1 |
| WATER | QS100 |
| GLYCERIN | 10 |
| COCO-BETAINE | 0,15 |
| CETRIMONIUM CHLORIDE | |
| CAPRYLYL/CAPRYL GLUCOSIDE | 0,6 |
| ASCORBIC ACID | 0,4 |

**Evaluation visuelle de la stabilité des compositions**

La stabilité de la composition colorante a été évaluée en observant la compositions à T0 (immédiatement après préparation de la composition) puis après 2 mois de stockage à température ambiante (25°C), et après 2 mois de stockage à 45°C.

| | Composition A3 selon l'invention |
|---|---|
| Observation à T0 (immédiatement après préparation) | Homogène (Pas de Déphasage) Texture : Gel lisse |
| Observation après 2 mois à 25°C | Homogène (Pas de Déphasage) Texture : Gel lisse |
| Observation après 2 mois à 45°C | Homogène (Pas de Déphasage) Texture : Gel lisse |

On observe que la composition selon l'invention est translucide et stable à température ambiante ainsi qu'à 45° pendant 2 mois.

La composition A3 a été mélangée avec 1 fois leur poids d'oxydant 20 volumes (6 g% ma en H2O2) ; le mélange ainsi obtenu a été appliqué sur des mèches de cheveux naturels à 90% blancs,

Le rapport de bain « mélange/mèche » est respectivement de 10/1 (g/g).

Le temps de pause de 30 minutes, sur plaque chauffante réglée à 27°C. A l'issue du temps de pause, les mèches sont rincées puis séchées sous casque à 40°C.

La couleur des mèches a été évaluée dans le système CIE L*a*b* au moyen d'un spectrocolorimètre Datacolor Spectraflash SF600X.

| | **L*** |
|---|---|
| Coloration obtenue avec le mélange composition A3+oxydant (invention) | **28,03** |

La composition A3 selon l'invention permet d'obtenir une coloration intense des fibres kératiniques.

***Exemple 4***

On a préparé la composition suivante à partir des ingrédients suivants dans les proportions suivantes indiquées en gramme :

| | Composition A4 selon l'invention |
|---|---|
| Ammonium hydroxide | 1,23 |
| Arginine | 3 |
| Ethanolamine | 5 |
| EDTA | 0,2 |
| Sodium Sulfite | 0,5 |
| Acide Citrique | 3,3 |
| Sodium Métasilicate | 2 |
| Toluène -2,5-diamine | 0,16 |
| 4-amino-2-hydroxytoluène | 0,92 |
| 5-Amino-6-Chloro-o-Crésol | 0,2 |
| 1-Hydroxyéthyl 4,5-diaminopyrazole sulfate | 1,44 |
| p-Aminophénol | 0,12 |
| Fragance | qs |
| Polyquaternium-11 | 1,84 |
| Hexadimethrine Chloride | 1,2 |
| Polyquaternium-6 | 0,8 |
| Cetylhydroxyéthylcellulose | 0,2 |
| Sclérotium gum | 1 |
| Eau | Qs 100 |
| Glycérine | 10 |
| Cétrimonium Chloride | 0,25 |
| Caprylyl/capryl glucoside | 0,6 |
| Acide ascorbique | 0,4 |

**Evaluation visuelle de la stabilité des compositions**

La stabilité de la composition colorante a été évaluée en les observant à T0 puis après 48h à température ambiante (25°C), puis après 2 mois de stockage à 45°C.

| | Composition A4 |
|---|---|
| Observation à T0 (immédiatement après préparation) à température ambiante (25°C) | Gel translucide Homogène (pas de déphasage) |
| Observation après 2 mois à 45°C | Gel translucide Homogène (pas de déphasage) |

On observe que la composition A4 selon l'invention est homogène et forment un gel translucide à T0. Après 2 mois à 45° la composition A4 selon l'invention est stable et sous forme de gel homogène et translucide.

La composition A4 a été mélangée avec 1 fois son poids d'oxydant 20 volumes (6 g% ma en H2O2). Le mélange ainsi obtenu a été appliqué sur des mèches de cheveux naturels à 90% blancs.

Le rapport de bain « mélange/mèche » est respectivement de 10/1 (g/g).

Le temps de pause de 30 minutes, sur plaque chauffante réglée à 27°C. A l'issue du temps de pause, les mèches sont rincées puis séchées sous casque à 40°C.

La couleur des mèches a été évaluée dans le système CIE L*a*b* au moyen d'un Spectrocolorimètre Datacolor Spectraflash SF600X

On obtient une coloration intense (L*=24.95) des fibres kératiniques.

## Revendications

1. Composition prête à l'emploi pour la coloration d'oxydation des fibres kératiniques en particulier des fibres kératiniques humaines telles que les cheveux, **caractérisée par le fait qu'**elle est obtenue par mélange

   d'une composition (A) comprenant

   - un ou plusieurs colorant(s) d'oxydation ;
   - un ou plusieurs gomme(s) de scléroglucane, en une teneur totale en poids supérieure ou égale à 0,5% par rapport au poids total de la composition ;
   - un ou plusieurs agent(s) alcalin(s) minéral(aux) ; et
   - un ou plusieurs agent(s) alcalin(s) organique(s) choisi(s) parmi les alcanolamine(s)

   et d'une composition oxydante (B) contenant au moins un agent oxydant chimique

2. Composition selon la revendication 1, **caractérisée en ce que** la ou les gommes de scléroglucane représentent de 0,5 à 10 % en poids, plus préférentiellement de 0,5 à 5 % en poids, et encore plus préférentiellement de 0,5 à 3 % en poids, mieux de 0.5% à 2% en poids et encore plus préférentiellement de 0,7 à 1,5 % en poids, par rapport au poids total de la composition (A)

3. Composition selon l'une quelconque des revendications précédentes, **caractérisée en ce que** le ou les colorants d'oxydation sont choisi(s) parmi les bases d'oxydation benzéniques de préférence choisies parmi les paraphénylènediamines, les bis-phénylalkylène-diamines, les para-aminophénols, les ortho-aminophénols, les bases hétérocycliques et leurs sels d'addition ; éventuellement combinées à un ou plusieurs coupleurs de préférence choisis parmi les méta-phénylènediamines, les méta-aminophénols, les méta-diphénols, les coupleurs naphthaléniques, et les coupleurs hétérocycliques ainsi que leurs sels d'addition.

4. Composition selon l'une quelconque des revendications précédentes **caractérisée en ce que** le ou les agent(s) alcalin(s) minéral(aux) sont choisis parmi l'ammoniaque (ou agents précurseurs d'ammoniac comme les sels d'ammonium, par exemple les halogénures d'ammonium et en particulier le chlorure d'ammonium), les silicates, les phosphates, les carbonates ou bicarbonates de métaux alcalins ou alcalino-terreux, tels que métasilicates de métaux alcalins ou alcalino-terreux, les carbonates ou bicarbonates de sodium ou de potassium, les hydroxydes de sodium ou de potassium, ou leurs mélanges.

5. Composition selon l'une quelconque des revendications précédentes **caractérisée en ce que** le ou les agent(s) alcalin(s) minéral(aux) est/sont présente(s) en une teneur totale allant de 0,1 à 10% en poids, plus préférentiellement de 0.5 à 8% en poids, mieux de 1 à 7% en poids par rapport au poids total de la composition (A).

6. Composition selon l'une quelconque des revendications précédentes **caractérisée en ce que** le ou les alcanolamines sont choisies parmi les mono-, di- ou tri-alcanolamines, comprenant un à trois radicaux hydroxyalkyle, identiques ou non, en $C_1$ à C4.

7. Composition selon l'une quelconque des revendications précédentes **caractérisée en ce que** le ou les alcanolamines sont choisies parmi la monoéthanolamine (MEA), la diéthanolamine, la triéthanolamine, la monoisopropanolamine, la diisopropanolamine, la N,N-diméthyléthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino-méthane, et leurs mélanges, de préférence la monoéthanolamine (MEA).

**8.** Composition selon la Composition selon l'une quelconque des revendications précédentes, **caractérisée en ce que** le ou les alcanolamines est/sont présente(s) en une teneur totale allant de préférence de 0,5 à 10% en poids, plus préférentiellement de 1 à 8% en poids, mieux de 2 à 8% en poids par rapport au poids total de la composition (A).

**9.** Composition selon l'une quelconque des revendications précédentes **caractérisée en ce que** le ou les agent(s) alcalin(s) minéral(aux) sont choisis parmi l'ammoniaque (hydroxyde d'ammonium) et de préférence **en ce que** la ou les alcanolamine(s), sont choisies parmi la monoethanolamine.

**10.** Composition selon l'une quelconque des revendications précédentes **caractérisée en ce qu'**elle comprend un ou plusieurs agents alcalins additionnels, différents desdits agents alcalins minéraux et desdites alcanolamines, de préférence choisis parmi les acides aminés basiques, de préférence l'histidine, la lysine, l'arginine, l'ornithine, la citrulline.

**11.** Composition selon l'une quelconque des revendications précédentes, **caractérisée en ce qu'**elle comprend un ou plusieurs polymère(s) associatif(s) non ioniques(s) ; de préférence choisis parmi les celluloses modifiées par des groupements comportant au moins une chaîne grasse, de préférence choisis parmi les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, et leurs mélanges, de préférence la cétylhydroxyethylcellulose.

**12.** Composition selon l'une quelconque des revendications précédentes, **caractérisée en ce qu'**elle comprend un ou plusieurs polymère(s) cationique(s) de préférence parmi :(1) les homopolymères de dialkyl diallyl ammonium, de préférence les homopolymères de diméthyldiallylammonium, ; et/ou

- (2) les polymères cationiques qui sont constitués de motifs récurrents répondant à la formule :

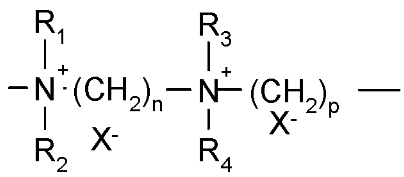
(IV)

dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique ; de préférence le polmyère composé de motifs formule (IV) pour lequel R1, R2, R3 et R4 représentent un radical méthyle, n=3, p=6 et X = Cl, dénommé Hexadimethrine chloride .

**13.** Composition selon l'une quelconque des revendications précédentes **caractérisée en ce que** le ou les agents oxydants chimique sont choisis parmi le peroxyde d'hydrogène et/ou un ou plusieurs système(s) générateur(s) de peroxyde d'hydrogène, de préférence parmi le peroxyde d'hydrogène.

**14.** Procédé de coloration des fibres kératiniques, comprenant l'application sur les fibres d'une composition colorante (A) telle que définie selon l'une quelconque des revendications 1 à 12, et d'une composition oxydante (B) comprenant au moins un agent oxydant chimique, de préférence choisi parmi le peroxyde d'hydrogène et/ou un ou plusieurs système(s) générateur(s) de peroxyde d'hydrogène, de préférence parmi le peroxyde d'hydrogène, la composition oxydante (B) étant mélangée avec la composition colorante (A) juste avant l'emploi (application sur lesdites fibres) (extemporanément) ou au moment de l'emploi, ou bien les compositions colorantes et oxydantes étant appliquées séquentiellement sans rinçage intermédiaire.

**Patentansprüche**

**1.** Gebrauchsfertige Zusammensetzung für die Oxidationsfärbung von Keratinfasern, insbesondere menschlichen Keratinfasern wie dem Haar, **dadurch gekennzeichnet, dass** sie durch Mischen einer Zusammensetzung (A), umfassend

- einen oder mehrere Oxidationsfarbstoffe;

- ein oder mehrere Skleroglucan-Gummen in einem Gesamtgewichtsgehalt größer oder gleich 0,5 %, bezogen auf das Gesamtgewicht der Zusammensetzung;
- ein oder mehrere mineralische alkalische Mittel und
- ein oder mehrere organische alkalische Mittel, ausgewählt aus Alkanolamin(en);

und einer oxidierenden Zusammensetzung (B), die mindestens ein chemisches Oxidationsmittel enthält; erhalten wird.

**2.** Zusammensetzung nach Anspruch 1, **dadurch gekennzeichnet, dass** das Skleroglucan-Gummi bzw. die Skleroglucan-Gummen 0,5 bis 10 Gew.-%, weiter bevorzugt 0,5 bis 5 Gew.-% und noch weiter bevorzugt 0,5 bis 3 Gew.-%, noch besser 0,5 bis 2 Gew.-% und noch weiter bevorzugt 0,7 bis 1,5 Gew.-%, bezogen auf das Gesamtgewicht der Zusammensetzung (A), ausmacht bzw. ausmachen.

**3.** Zusammensetzung nach einem der vorhergehenden Ansprüche, **dadurch gekennzeichnet, dass** der Oxidationsfarbstoff bzw. die Oxidationsfarbstoffe aus Benzol-Oxidationsbasen, vorzugsweise ausgewählt aus para-Phenylendiaminen, Bisphenylalkylendiaminen, para-Aminophenolen, ortho-Aminophenolen, heterocyclischen Basen und Additionssalzen davon ausgewählt ist bzw. sind; gegebenenfalls in Kombination mit einem oder mehreren Kupplern, vorzugsweise ausgewählt aus meta-Phenylendiaminen, meta-Aminophenolen, meta-Diphenolen, Naphthalin-Kupplern und heterocyclischen Kupplern sowie Additionssalzen davon.

**4.** Zusammensetzung nach einem der vorhergehenden Ansprüche, **dadurch gekennzeichnet, dass** das mineralische alkalische Mittel bzw. die mineralischen alkalischen Mittel aus wässrigem Ammoniak (oder Ammoniak-Vorläufern wie Ammoniumsalzen, beispielsweise Ammoniumhalogeniden und insbesondere Ammoniumchlorid), Alkali- oder Erdalkalimetallsilicaten, -phosphaten, -carbonaten oder -hydrogencarbonaten, wie Alkali- oder Erdalkalimetallmetasilicaten, Natrium- oder Kaliumcarbonat oder -Hydrogencarbonat, Natrium- oder Kaliumhydroxid oder Mischungen davon ausgewählt ist bzw. sind.

**5.** Zusammensetzung nach einem der vorhergehenden Ansprüche, **dadurch gekennzeichnet, dass** das mineralische alkalische Mittel bzw. die mineralischen alkalischen Mittel in einem Gesamtgehalt im Bereich von 0,1 bis 10 Gew.-%, weiter bevorzugt von 0,5 bis 8 Gew.-%, noch besser von 1 bis 7 Gew.-%, bezogen auf das Gesamtgewicht der Zusammensetzung (A), vorliegt bzw. vorliegen.

**6.** Zusammensetzung nach einem der vorhergehenden Ansprüche, **dadurch gekennzeichnet, dass** das Alkanolamin bzw. die Alkanolamine aus Mono-, Di- oder Trialkanolaminen mit einem bis drei gleichen oder verschiedenen $C_1$- bis $C_4$-Hydroxyalkylresten ausgewählt ist bzw. sind.

**7.** Zusammensetzung nach einem der vorhergehenden Ansprüche, **dadurch gekennzeichnet, dass** das Alkanolamin bzw. die Alkanolamine aus Monoethanolamin (MEA), Diethanolamin, Triethanolamin, Monoisopropanolamin, Diisopropanolamin, N,N-Dimethylethanolamin, 2-Amino-2-methyl-1-propanol, Triisopropanolamin, 2-Amino-2-methyl-1,3-propandiol, 3-Amino-1,2-propandiol, 3-Dimethylamino-1,2-propandiol, Tris(hydroxymethylamino)methan und Mischungen davon, vorzugsweise Monoethanolamin (MEA), ausgewählt ist bzw. sind.

**8.** Zusammensetzung gemäß der Zusammensetzung nach einem der vorhergehenden Ansprüche, **dadurch gekennzeichnet, dass** das Alkanolamin bzw. die Alkanolamine in einem Gesamtgehalt im Bereich von vorzugsweise 0,5 bis 10 Gew.-%, weiter bevorzugt 1 bis 8 Gew.-%, noch besser 2 bis 8 Gew.-%, bezogen auf das Gesamtgewicht der Zusammensetzung (A), vorliegt bzw. vorliegen.

**9.** Zusammensetzung nach einem der vorhergehenden Ansprüche, **dadurch gekennzeichnet, dass** das mineralische alkalische Mittel bzw. die mineralischen alkalischen Mittel aus wässrigem Ammoniak (Ammoniumhydroxid) ausgewählt ist bzw. sind und vorzugsweise das mineralische alkalische Mittel bzw. die mineralischen alkalischen Mittel aus Monoethanolamin ausgewählt ist bzw. sind.

**10.** Zusammensetzung nach einem der vorhergehenden Ansprüche, **dadurch gekennzeichnet, dass** sie ein oder mehrere zusätzliche alkalische Mittel, die von den mineralischen alkalischen Mitteln und den Alkanolaminen verschieden sind, umfasst, vorzugsweise ausgewählt aus basischen Aminosäuren, vorzugsweise Histidin, Lysin, Arginin, Ornithin und Citrullin.

**11.** Zusammensetzung nach einem der vorhergehenden Ansprüche, **dadurch gekennzeichnet, dass** sie ein oder

mehrere nichtionische assoziative Polymere umfasst; vorzugsweise ausgewählt aus Cellulosen, die durch Gruppen mit mindestens einer Fettkette modifiziert sind, vorzugsweise ausgewählt aus Hydroxyethylcellulosen, die durch Gruppen mit mindestens einer Fettkette wie Alkyl-, Arylalkyl- oder Alkylarylgruppen oder Mischungen davon modifiziert sind, wobei es sich bei den Alkylgruppen vorzugsweise um $C_8$-$C_{22}$-Alkylgruppen handelt, und Mischungen davon, vorzugsweise Cetylhydroxypropylcellulose.

**12.** Zusammensetzung nach einem der vorhergehenden Ansprüche, **dadurch gekennzeichnet, dass** sie ein oder mehrere kationische Polymere umfasst, vorzugsweise aus:

- (1) Dialkyldiallylammonium-Homopolymeren, vorzugsweise Dimethyldiallylammonium-Homopolymeren, und/oder
- (2) kationischen Polymeren, die aus Wiederholungseinheiten aufgebaut sind, die der Formel:

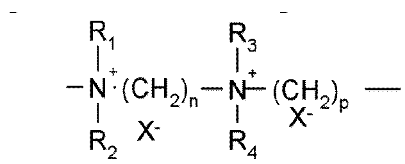

(IV)

entsprechen, wobei R1, R2, R3 und R4 gleich oder verschieden sind und für einen Alkyl- oder Hydroxyalkylrest mit 1 bis 4 Kohlenstoffatomen stehen, n und p ganze Zahlen im Bereich von 2 bis 20 sind und X- ein Anion ist, das sich von einer Mineralsäure oder organischen Säure ableitet; vorzugsweise dem Polymer, das aus Einheiten der Formel (IV) aufgebaut ist, wobei R1, R2, R3 und R4 für einen Methylrest stehen, n = 3, p = 6 und X = Cl, mit der Bezeichnung Hexadimethrinchlorid.

**13.** Zusammensetzung nach einem der vorhergehenden Ansprüche, **dadurch gekennzeichnet, dass** das chemische Oxidationsmittel bzw. die chemischen Oxidationsmittel aus Wasserstoffperoxid und/oder einem oder mehreren Wasserstoffperoxid erzeugenden Systemen, vorzugsweise aus Wasserstoffperoxid, ausgewählt ist bzw. sind.

**14.** Verfahren zum Färben von Keratinfasern, bei dem man auf die Fasern eine Färbezusammensetzung (A) gemäß einem der Ansprüche 1 bis 12 und eine oxidierende Zusammensetzung (B), umfassend mindestens ein chemisches Oxidationsmittel, vorzugsweise ausgewählt aus Wasserstoffperoxid und/oder einem oder mehreren Wasserstoffperoxid erzeugenden Systemen, vorzugsweise aus Wasserstoffperoxid, aufbringt, wobei die oxidierende Zusammensetzung (B) kurz vor der Anwendung (Aufbringen auf die Fasern) (extemporal) oder zum Zeitpunkt der Anwendung mit der Färbezusammensetzung (A) gemischt wird oder auch die Färbezusammensetzung und die oxidierende Zusammensetzung ohne Zwischenspülung nacheinander aufgebracht werden.

## Claims

**1.** Ready-to-use composition for the oxidation dyeing of keratin fibres, in particular human keratin fibres such as the hair, **characterized in that** it is obtained by mixing

a composition (A) comprising:

- one or more oxidation dyes;
- one or more scleroglucan gums in a total weight content of greater than or equal to 0.5% relative to the total weight of the composition;
- one or more mineral alkaline agents; and
- one or more organic alkaline agents chosen from alkanolamine(s);

and an oxidizing composition (B) containing at least one chemical oxidizing agent.

**2.** Composition according to Claim 1, **characterized in that** the scleroglucan gum(s) represent from 0.5% to 10% by weight, more preferentially from 0.5% to 5% by weight, and even more preferentially from 0.5% to 3% by weight, better still from 0.5% to 2% by weight and even more preferentially from 0.7% to 1.5% by weight, relative to the total weight of composition (A).

**3.** Composition according to either of the preceding claims, **characterized in that** the oxidation dye(s) are chosen from benzene-based oxidation bases, preferably chosen from para-phenylenediamines, bis(phenyl)alkylenediamines, para-aminophenols, ortho-aminophenols, heterocyclic bases and the addition salts thereof, optionally combined with one or more couplers, preferably chosen from meta-phenylenediamines, meta-aminophenols, meta-diphenols, naphthalene-based couplers, and heterocyclic couplers, and also the addition salts thereof.

**4.** Composition according to any one of the preceding claims, **characterized in that** the mineral alkaline agent(s) is/are chosen from aqueous ammonia (or ammonia precursors such as ammonium salts, for example ammonium halides and in particular ammonium chloride), alkali metal or alkaline-earth metal silicates, phosphates, carbonates or bicarbonates, such as alkali metal or alkaline-earth metal metasilicates, sodium or potassium carbonate or bicarbonate, sodium or potassium hydroxide, or mixtures thereof.

**5.** Composition according to any one of the preceding claims, **characterized in that** the mineral alkaline agent(s) is/are present in a total content ranging from 0.1% to 10% by weight, more preferentially from 0.5% to 8% by weight and better still from 1% to 7% by weight, relative to the total weight of the composition (A).

**6.** Composition according to any one of the preceding claims, **characterized in that** the alkanolamine(s) is/are chosen from monoalkanolamines, dialkanolamines or trialkanolamines, comprising one to three identical or different $C_1$ to $C_4$ hydroxyalkyl radicals.

**7.** Composition according to any one of the preceding claims, **characterized in that** the alkanolamine(s) is/are chosen from monoethanolamine (MEA), diethanolamine, triethanolamine, monoisopropanolamine, diisopropanolamine, N,N-dimethylethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethyl)aminomethane, and mixtures thereof, preferably monoethanolamine (MEA).

**8.** Composition according to the composition according to any one of the preceding claims, **characterized in that** the alkanolamine(s) is/are present in a total content preferably ranging from 0.5% to 10% by weight, more preferentially from 1% to 8% by weight and better still from 2% to 8% by weight, relative to the total weight of the composition (A).

**9.** Composition according to any one of the preceding claims, **characterized in that** the mineral alkaline agent(s) is/are chosen from aqueous ammonia (ammonium hydroxide) and preferably **in that** the alkanolamine(s) is/are chosen from monoethanolamine.

**10.** Composition according to any one of the preceding claims, **characterized in that** it comprises one or more additional alkaline agents other than said mineral alkaline agents and said alkanolamines, preferably chosen from basic amino acids, preferably histidine, lysine, arginine, ornithine and citrulline.

**11.** Composition according to any one of the preceding claims, **characterized in that** it comprises one or more nonionic associative polymers; preferably chosen from celluloses modified with groups including at least one fatty chain, preferably chosen from hydroxyethylcelluloses modified with groups including at least one fatty chain such as alkyl, arylalkyl or alkylaryl groups, or mixtures thereof, and in which the alkyl groups are preferably $C_8$-$C_{22}$, and mixtures thereof, preferably cetylhydroxyethylcellulose.

**12.** Composition according to any one of the preceding claims, **characterized in that** it comprises one or more cationic polymers preferably from:

- (1) dialkyldiallylammonium homopolymers, preferably dimethyldiallylammonium homopolymers; and/or
- (2) cationic polymers that are constituted of repeating units corresponding to the formula:

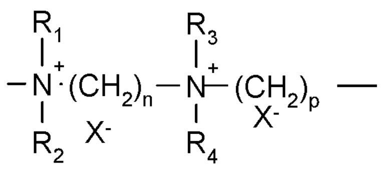

(IV)

in which R1, R2, R3 and R4, which may be identical or different, denote an alkyl or hydroxyalkyl radical containing

from 1 to 4 carbon atoms, n and p are integers ranging from 2 to 20, and X- is an anion derived from a mineral or organic acid; preferably, the polymer composed of units of formula (IV) for which R1, R2, R3 and R4 represent a methyl radical, n = 3, p = 6 and X = Cl, known as hexadimethrine chloride.

**13.** Composition according to any one of the preceding claims, **characterized in that** the chemical oxidizing agent(s) are chosen from hydrogen peroxide and/or one or more systems for generating hydrogen peroxide, preferably from hydrogen peroxide.

**14.** A process for dyeing keratin fibres, comprising the application to the fibres of a dye composition (A) as defined in any one of Claims 1 to 12, and of an oxidizing composition (B) comprising at least one chemical oxidizing agent, preferably chosen from hydrogen peroxide and/or one or more systems for generating hydrogen peroxide, preferably from hydrogen peroxide, the oxidizing composition (B) being mixed with the dye composition (A) just before use (application to said fibres) (extemporaneously) or at the time of use, or alternatively the dye composition and oxidizing composition being applied sequentially without intermediate rinsing.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 2010192969 A1, DEGEORGE MICHAEL
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A
- FR 2633940